# EUROPÄISCHE PATENTSCHRIFT

(11) **EP 0 536 085 B1**
(45) Veröffentlichungstag und Bekanntmachung des Hinweises auf die Patenterteilung: **11.11.1998**
(21) Anmeldenummer: 92810727.5
(22) Anmeldetag: 24.09.1992
(51) Int. Cl.: C08G 59/20, C08L 63/00, C09D 163/00

(54) **Feste Zusammensetzungen aus Polyglycidylverbindungen eines Molekulargewichts unter 1500**
Solid compositions based on polyglycidyl compounds having a molecular weight lower than 1500
Compositions solides à base de composés polyglycidyliques ayant un poids moléculaire inférieure à 1500

(30) Priorität: 03.10.1991 CH 2921/91
(43) Veröffentlichungstag der Anmeldung: 07.04.1993
(73) Patentinhaber: Ciba Specialty Chemicals Holding Inc., 4057 Basel (CH)
(72) Erfinder: Cotting, Jacques-Alain, Dr., CH-1729 Bonnefontaine (CH); Gottis, Philippe-Guilhaume, Dr., F-68200 Mulhouse (FR)

(56) Entgegenhaltungen:
- EP-A- 0 006 535
- EP-A- 0 018 950
- DE-A- 2 602 221
- DE-B- 1 158 435
- GB-A- 1 073 633
- DATABASE WPI Derwent Publications Ltd., London, GB; AN 85-135281 & DD-A-218 376 (VEB LEUNA-WERK ULBRICHT)

## Beschreibung

Die vorliegende Erfindung betrifft eine feste Zusammensetzung aus Polyglycidylverbindungen eines Molekulargewichts unter 1500 als wesentlichen Komponenten, ein Verfahren zur Herstellung dieser Zusammensetzungen, deren Verwendung und einen Pulverlack mit diesen Zusammensetzungen.

Polyglycidylverbindungen werden heute vielfach als reaktiver Bestandteil härtbarer Zusammensetzungen eingesetzt, beispielsweise als Härter bzw. Vernetzungsmittel in Pulverlackzusammensetzungen auf Basis von Polyestern. Viele Polyglycidylverbindungen haben dabei den Nachteil, dass sie entweder als solche bei Raumtemperatur oder mässig hoher Temperatur flüssig sind oder mit vertretbarem Aufwand nur in einer Form gewonnen werden können, in der sie aufgrund von Verunreinigungen in einer bei den genannten Temperaturen flüssigen oder halbfesten Form vorliegen. Die homogene Einarbeitung dieser flüssigen Verbindungen in feste Zusammensetzungen erfordert in der Praxis einen beträchtlich höheren verfahrenstechnischen Aufwand, als er notwendig ist, wenn nur feste Glycidylverbindungen eingesetzt werden.

Den Hauptanteil der bekannten festen Polyglycidylverbindungen bilden aber Diglycidylverbindungen. Diese haben wiederum andere Nachteile, wenn sie als einziges Vernetzungsmittel für härtbare Zusammensetzungen eingesetzt werden. Beispielsweise sind Diglycidylverbindungen im allgemeinen weniger reaktiv als höherfunktionelle Polyglycidylverbindungen und ergeben einen geringeren Vernetzungsgrad. Entsprechend eingeschränkt sind ihre Einsatzmöglichkeiten.

Einige wenige höherfunktionelle Polyglycidylverbindungen, z. B. Trimesinsäuretriglycidylester oder insbesondere Triglycidylisocyanurat, können zwar auch in fester Form gewonnen werden, hierzu sind jedoch entweder unübliche und teure Verfahren oder ein relativ grosser und somit ebenfalls teuerer Reinigungsaufwand erforderlich. Immerhin hat jedoch das feste Triglycidylisocyanurat auf manchen Einsatzgebieten für Glycidylhärter eine einzigartige Stellung erringen können, obwohl einige flüssige oder halbfeste Glycidylverbindungen bekannt geworden sind, die ihm als Vernetzungsmittel im Hinblick auf die Eigenschaften der fertiggestellten Zusammensetzungen oder daraus erhaltener gehärteter Produkte gleichkommen oder es sogar übertreffen. Flüssige Polyglycidylverbindungen dieser Art sind beispielsweise in der EP-A-0 506 617 und in der EP-A-0 383 601 (veröffentlicht am 22. 08. 1990) beschrieben.

Um auch Polyglycidylverbindungen, die in flüssiger Form vorliegen, in einfacher Weise als Härter für feste Zusammensetzungen verwenden zu können, ist im Stand der Technik schon vorgeschlagen worden, sie durch Vermischen mit bestimmten anderen Substanzen zu verfestigen. Beispielsweise ist in der EP-A-0 178 705 (veröffentlicht am 23. April 1986) eine feste Zusammensetzung beschrieben, die aus stark unreinem und deshalb in flüssiger Form vorliegendem Triglycidylisocyanurat und einem nicht reaktiven, leicht kristallisierbaren Polyester besteht und sich als Härter für carboxylterminierte Polyester eignet. Aus der EP-A-0 462 053 ist ausserdem eine feste Zusammensetzung aus flüssigen oder halbfesten Glycidylverbindungen und einem festen Kondensationspolymer mit grosser Porenfläche bekannt, das aus Harnstoff oder Melamin und Formaldehyd hergestellt wird. Die genannten Zusammensetzungen haben als Härtungsmittel den Nachteil, dass sie relativ grosse Mengen inerter, d. h. selbst nicht härtend wirkender Stoffe enthalten. Dies führt beispielsweise zu einem unnötig niedrigen Epoxidgehalt bei diesen Härtern und macht daher die Verwendung relativ grosser Mengen Härter erforderlich, die unter anderem zu höheren Kosten führt.

Aufgabe der vorliegenden Erfindung ist es. neue feste Massen auf Basis von normalerweise in flüssiger Form vorliegenden Polyglycidylverbindungen zur Verfügung zu stellen, die im wesentlichen keine inerten Komponenten enthalten.

Überraschend hat es sich gezeigt, dass in vielen Fällen niedrigmolekulare feste Polyglycidylverbindungen andere Polyglycidylverbindungen ähnlichen Molekulargewichts, die aber normalerweise in flüssiger Form vorliegen, in grossen Mengen aufnehmen oder in sich löscn können, wobei nicht klebrige, feste Massen entstehen.

Gegenstand der vorliegenden Erfindung ist daher eine feste Zusammensetzung aus Polyglycidylverbindungen eines Molekulargewichts unter 1500, mit einem Epoxidgehalt von über 3,5 Äquivalenten pro Kilogramm Verbindung, die aus einer oder mehreren festen Polyglycidylverbindungen und insgesamt nicht weniger als 5 Gewichtsprozent einer normalerweise in flüssiger Form vorliegenden Polyglycidylverbindung oder eines Gemisches von normalerweise in flüssiger Form vorliegenden Polyglycidylverbindungen als wesentlichen Komponenten besteht, wobei sich die Gehaltsangabe auf die Gesamtmenge aller Polyglycidylverbindungen in der Zusammensetzung bezieht und die Zusammensetzung die festen Polyglycidylverbindungen oder zumindest einen Teil der festen Polyglycidylverbindungen in Form einer oder mehr als einer festen Mischphase enthält und die feste Mischphase oder die festen Mischphasen im wesentlichen die Gesamtmenge der normalerweise in flüssiger Form vorliegenden Polyglycidylverbindungen als zusätzliche Komponente oder Komponenten enthält oder enthalten.

Die erfindungsgemässen Zusammensetzungen sind praktisch nicht klebrig.

Bevorzugt handelt es sich bei den erfindungsgemässen Zusammensetzungen um feste, kristalline Mischphasen (feste Lösungen) aus einer festen und einer weiteren Polyglycidylverbindung, die normalerweise in einer flüssigen Form vorliegt. Das Vorhandensein einer festen Lösung kann z. B. mit Hilfe der Röntgendiffraktometrie festgestellt werden, da sich die Lagen der Reflexe im Röntgendiffraktogramm einer solchen Zusammensetzung im allgemeinen nur unwesentlich von denen im Diffraktogramm der verwendeten reinen festen Polyglycidylverbindung unterscheiden.

Der Begriff "Polyglycidylverbindungen" umfasst im vorliegenden Zusammenhang Verbindungen, die unsubstituierte Glycidylgruppen aufweisen, aber auch Verbindungen, die z. B. mit Methylgruppen substituierte Glycidylgruppen aufweisen. Die Polyglycidylverbindungen in den erfindungsgemässen Zusammensetzungen haben bevorzugt ein Molekulargewicht zwischen 200 und 1200, insbesondere zwischen 200 und 1000. Ihr Epoxidgehalt sollte im allgemeinen über 3,5 Äquivalenten pro Kilogramm der Verbindung liegen, günstigerweise bei mindestens 4 Äquivalenten, besonders bevorzugt bei 5 und mehr Äquivalenten pro Kilogramm.

Als feste Polyglycidylverbindungen kommen Verbindungen mit Schmelzpunkten oberhalb von Raumtemperatur (ca. 25 °C) bis etwa 250 °C in Frage. Bevorzugt liegen die Schmelzpunkte der Verbindungen aber im Bereich von 60 bis 150 °C. Selbstverständlich müssen die Schmelzpunkte nicht ganz scharf sein, wie dies beispielsweise von verunreinigten Verbindungen allgemein bekannt ist.

Mit "Polyglycidylverbindungen, die normalerweise in einer flüssigen Form vorliegen" sind Polyglycidylverbindungen gemeint, die bei Raumtemperatur, d. h. bei ca. 15 bis 25 °C, oder bei nur leicht erhöhter Temperatur, z. B. bei 30 oder 40 °C, in einer schon merklich fliessfähigen Form vorliegen. Dieser Begriff umfasst reine Polyglycidylverbindungen, deren Schmelzpunkt unterhalb der genannten Temperaturen liegt, aber auch solche Formen von Polyglycidylverbindungen, die zwar hochrein bei den genannten Temperaturen fest wären, die aber in der Form, von der bei der Herstellung der erfindungsgemässen Zusammensetzungen wirklich ausgegangen wird, noch flüssig sind. Dabei kann es sich z. B. um technische Rohformen bestimmter Polyglycidylverbindungen handeln. Beispielsweise kann Triglycidylisocyanurat, das im Verlauf seiner Herstellung oftmals zunächst als Öl anfällt, in dieser Form als flüssige Polyglycidylkomponente für die erfindungsgemässen Zusammensetzungen eingesetzt werden. Ebenso gilt dies beispielsweise für Trimesinsäuretriglycidylester, der zwar ausgehend von dem teuren und nur schwer verfügbaren Glycidol als einer Komponente relativ leicht in fester Form erhältlich ist, bei der für die technische Herstellung von Glycidylestern üblichen Umsetzung der entsprechenden Säuren mit Epichlorhydrin aber nur in einer verunreinigten, bei Raumtemperatur fliessfähigen Form erhalten werden kann.

Die normalerweise in einer flüssigen Form vorliegenden Polyglycidylverbindungen weisen im allgemeinen und vorzugsweise eine höhere Funktionalität als die festen Polyglycidylverbindungen und z. B. mindestens drei Glycidylgruppen pro Molekül auf. Ihr Epoxidgehalt liegt bevorzugt bei 5,5 und mehr Äquivalenten Epoxidgruppen pro Kilogramm der Verbindung.

Bevorzugt bestehen die erfindungsgemässen Zusammensetzungen aus Polyglycidylverbindungen, die Glycidylether- und/oder Glycidylestergruppen aufweisen. Eine Polyglycidylverbindung kann dabei auch beide Arten von Glycidylgruppen enthalten, wie z. B. 4-Glycidyloxy-benzoesäureglycidylester, der gemäss der Erfindung als feste Polyglycidylverbindung verwendet werden kann.

Weiterhin besonders bevorzugt als feste Polyglycidylverbindungen sind Diglycidylester und/oder Diglycidylether.

Die Diglycidylester leiten sich z. B. von aromatischen, araliphatischen, cycloaliphatischen, heterocyclischen, heterocyclisch-aliphatischen und heterocyclisch-aromatischen Dicarbonsäuren mit 6 bis 20, insbesondere 6 bis 12 Ringkohlenstoffatomen oder von aliphatischen Dicarbonsäuren mit 2 bis 10 Kohlenstoffatomen ab. Verbindungen dieses Typs sind allgemein bekannt und z. B. im U.S. Patent US-A-3,859,314 oder in der DE-A-31 26 411 (veröffentlicht am 13. Januar 1983) beschrieben. Beispiele für geeignete Dicarbonsäuren sind: Phthalsäure, Isophthalsäure, Terephthalsäure, 2,5-Dimethylphthalsäure, Naphtalin-2,6-dicarbonsäure, Naphtalin- 1,8-dicarbonsäure, Naphtalin-2,3-dicarbonsäure, Diphenylether-4,4'-dicarbonsäure, Diphenyl-2,2'-dicarbonsäure, Tetrachlorphthalsäure, 2,5-Dichlorphthalsäure, o-, m- oder p-Phenylendiessigsäure, Oxalsäure, Malonsäure, Bernsteinsäure, Adipinsäure, 2,2,4-Trimethyladipinsäure, 2,4,4-Trimethyladipinsäure, Sebazinsäure, Azelainsäure, Fumarsäure, Maleinsäure und die durch Anlagerung von Acrylnitril oder Acrylsäureester an Verbindungen mit aktivierbaren Wasserstoffatomen, wie Ketone, Stickstoffverbindungen, Diole oder Dithiole, erhaltbaren Dicarbonsäuren, Tetrahydrophthalsäure, Methyltetrahydrophthalsäure, Hexahydrophthalsäure, Methylhexahydrophthalsäure, Endomethylen-hexahydrophthalsäure, Hexahydroterephthalsäure, Hexahydroisophthalsäure, Thiophen-2,5-dicarbonsäure, Furan-2,5-dicarbonsäure, Furan-3,4-dicarbonsäure, Pyrazin-3,4-dicarbonsäure, unsubstituiertes oder in 5-Stellung alkylsubstituiertes 1,3-Bis-(carboxyethyl)-hydantoin, 1,1-Methylen-bis-[3-(p-glycidyloxycarbonylbenzyl)-5,5-dimethylhydantoin] sowie andere einen oder mehrere Hydantoinring enthaltende Dicarbonsäureester und N,N'-Bis-(p-glycidyloxycarbonylbenzoyl)-isophorondiamin.

Ganz besonders bevorzugt sind Zusammensetzungen, die als Diglycidylester Terephthalsäurediglycidylester oder Isophthalsäurediglycidylester oder Trans-hexahydrophthalsäurediglycidylester oder Oxalsäurediglycidylester oder Adipinsäurediglycidylester oder Sebazinsäurediglycidylester oder Azelainsäurediglycidylester oder Bernsteinsäurediglycidylester enthalten.

Als feste Polyglycidylether sind z. B. Verbindungen mit glycidylisierten aromatischen Hydroxylgruppen geeignet, insbesondere die Diglycidylether, abgeleitet von 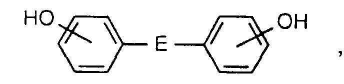 insbesondere von 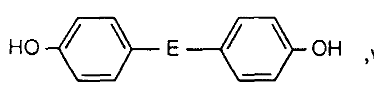 worin E jedesmal für -CH₂-, -C(CH₃)₂-, -O-, -S-, -SO- oder insbesondere für -SO₂- steht.

Eine weitere spezielle Ausführungsform der oben beschriebenen Zusammensetzungen enthält als feste Polyglycidylverbindung einen Diglycidylether der Formel (I): 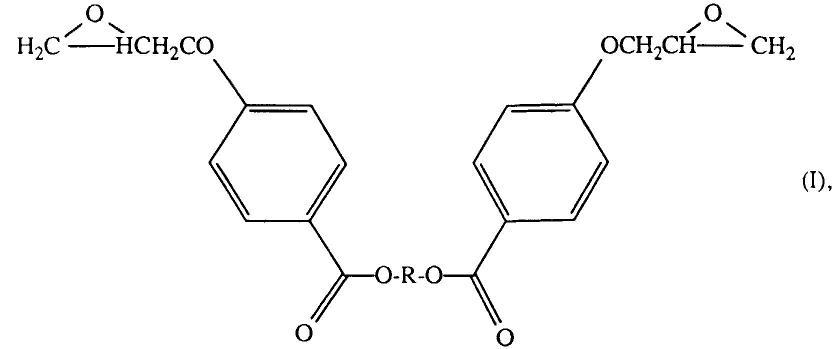 worin R ein organisches Diradikal mit 2 bis 15 Kohlenstoffatomen bedeutet.

Bevorzugt handelt es sich bei R um Diradikale, die sich von Diolen R(OH)₂ ableiten, die auch als Ausgangsstoffe für Polyester üblich sind, beispielsweise um 1,2-Ethandiyl, 1,2-Propandiyl, 1,3-Butandiyl und 1,4-Butandiyl, 2,2-Dimethyl- 1,3-propandiyl, 1,6-Hexandiyl, 2-Ethyl-1,3-hexandiyl oder Gruppen der Formeln: 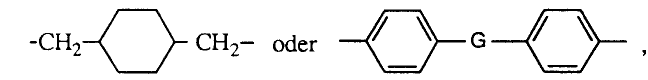 worin G für -CH₂-, -C(CH₃)₂-, -O-, -S-, -SO- oder -SO₂- steht. Besonders bevorzugte Reste R sind 1,3-Butandiyl, 1,6-Hexandiyl, 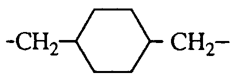 und

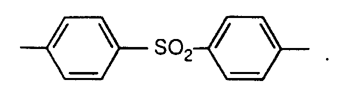

Die genannten Verbindungen, die teilweise neu sind, sind nach üblichen Methoden herstellbar, indem man z. B. 4-Hydroxy-benzoesäuremethylester mit dem entsprechenden Diol R(OH)₂ umestert und anschliessend mit Epichlorhydrin glycidylisiert. Eine weiteres ähnliches Darstellungsverfahren ist von H. KAKIUCHI und S. TAKEI in "New Epoxy Resins from Alkylene-bis-(p-hydroxy benzoate)", Org. Coat. Plast. Chem. 1979, 40, 899 - 902 beschrieben.

Die normalerweise in einer flüssigen Form vorliegenden Polyglycidylverbindungen umfassen insbesondere Polyglycidylether und -ester mit drei oder mehr Glycidylgruppen pro Molekül, z. B. Polyglycidylester von Benzolpolycarbonsäuren, wie beispielsweise 1,2,3-Benzoltricarbonsäure (Hemimellitsäure), 1,2,4-Benzoltricarbonsäure (Trimellitsäure), 1,3,5-Benzoltricarbonsäure (Trimesinsäure), 1,2,3,4-Benzoltetracarbonsäure (Mellophansäure), 1,2,3,5-Benzoltetracarbonsäure, Benzolpentacarbonsäure und Benzolhexacarbonsäure (Mellitsäure), Naphthalintetracarbonsäure, Perylentetracarbonsäure oder Tetracarbonsäuren der Formel 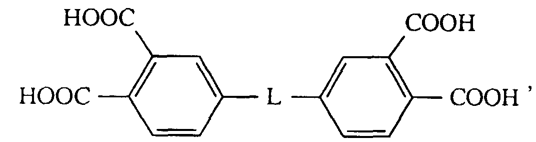 worin L für -CH₂-, -C(CH₃)₂-, -O-, -S-, -SO- oder -SO₂- steht, insbesondere Benzophenon-3,3',4,4'-tetracarbonsäure. Die Herstellung solcher Polyglycidylester kann nach bekannten Methoden, z. B. gemäss den bei den Diglycidylestern zitierten Druckschriften, erfolgen.

Besonders bevorzugt sind Zusammensetzungen, die Polyglycidylester mit mindestens drei Glycidylgruppen pro Molekül als normalerweise in einer flüssigen Form vorliegende Polyglycidylverbindungen enthalten, z. B. Polyglycidylester mit drei oder vier Glycidylgruppen, insbesondere ausgewählt aus nicht festen Formen von Trimellitsäuretriglycidylester, Trimesinsäuretriglycidylester und Pyromellitsäuretetraglycidylester.

Als flüssige Glycidylester in erfindungsgemässen Zusammensetzungen kommen auch die Verbindungen der Formeln (II) oder (III) in Frage: 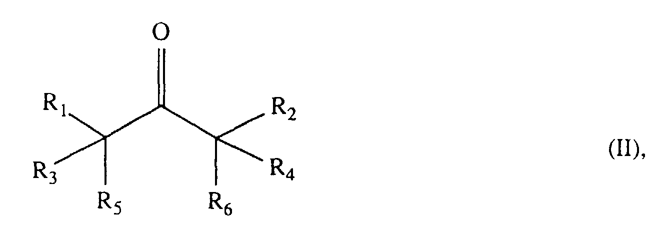 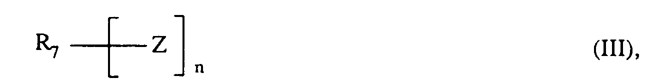 wobei in Formel (II) R₁, R₂, R₃ und R₄ unabhängig voneinander Wasserstoff, C₁-C₄-Alkyl oder Reste der Formel (IV) bedeuten: 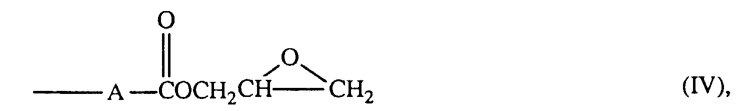 worin A für eine Polymethylengruppe mit 2 bis 4 Kohlenstoffatomen steht, und R₅ und R₆ unabhängig voneinander Wasserstoff, C₁-C₄-Alkyl, Reste der Formel (IV) oder zusammen eine unsubstituierte oder eine mit C₁-C₄-Alkyl substituierte Methylen- oder Polymethylengruppe mit 2 bis 7 Kohlenstoffatomen bedeuten,
und in Formel (III) n eine ganze Zahl von 2 bis 6, R₇ einen n-wertigen organischen Rest, und Z gleiche oder unterschiedliche Reste der Formel (V) bedeuten: 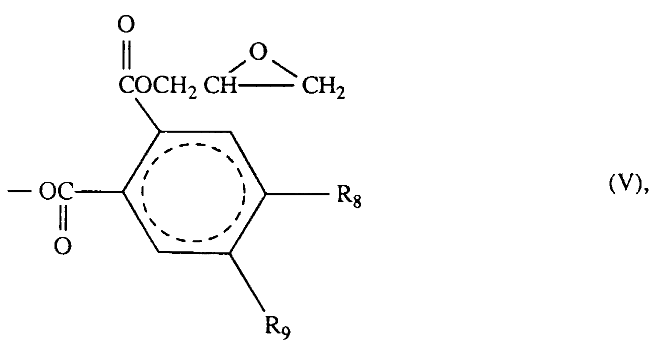 worin R₈ und R₉ entweder unabhängig voneinander Wasserstoff, Chlor, Brom oder C₁-C₄-Alkyl oder eines von beiden einen Rest der Formel (VI): 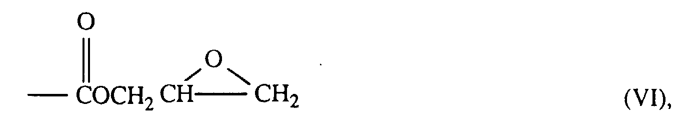 und das andere Wasserstoff, Chlor, Brom oder C₁ -C₄-Alkyl bedeuten, und der sechsgliedrige Ring in Formel (V) aromatisch oder nicht aromatisch ist.

Bevorzugt beträgt die Zahl der Glycidylgruppen pro Molekül der genannten Verbindungen hierbei ebenfalls mindestens 3.

In der Formel (II) bedeuten R₁ bis R₄ vorzugsweise einen Rest der Formel (IV) und R₅ und R₆ vorzugsweise zusammen eine unsubstituierte oder mit C₁-C₄-Alkyl substituierte Methylen- oder Polymethylengruppe mit 2 bis 7 Kohlenstoffatomen, insbesondere eine unsubstituierte Polymethylengruppe mit 2 bis 4 Kohlenstoffatomen. Die Zahl der Alkylsubstituenten kann zwar bis zum Doppelten der Zahl der Kohlenstoffatome der Methylen- oder Polymethylengruppe betragen, sollte aber zweckmässig nur 1 bis 2 betragen. In Formel (IV) ist A bevorzugt eine Ethylengruppe. Besonders geeignete Verbindungen der Formel (II) sind beispielsweise 2,2,5,5-Tetra-(β-carboxyethyl)-cyclopentanontetraglycidylester, 2,2,6,6-Tetra-(β-carboxyethyl)-cyclohexanontetraglycidylester und der 2,2,4,4-Tetra-(β-carboxyethyl)-pentanon-(3)-tetraglycidylester oder 1, 1,3,3-Tetra-(β-carboxyethyl)-acetontetraglycidylester geeignet.

Die Verbindungen der Formel (II) sind z. B. aus den entsprechenden Polycarbonsäuren erhältlich, beispielsweise durch Umsetzung der Carbonsäuren mit Epihalogenhydrin zu den Halogenhydrinestern, wobei Halogen vorzugsweise Brom und insbesondere Chlor bedeutet. Die Halogenhydrinester können danach mit halogenwasserstoffbindenden Mitteln zu den entsprechenden Glycidylestern dehydrohalogeniert werden, wie z. B. in der DE-A-23 19 815 (= GB 1,409,835), veröffentlicht am 22. November 1973, näher beschrieben. Die Herstellung der als Ausgangsprodukte benötigten cycloaliphatischen Polycarbonsäuren kann analog dem britischen Patent Nr. 1,033,697 (= US 3,344,117), veröffentlicht am 22. Juni 1966, erfolgen, die der aliphatischen Polycarbonsäuren z. B. analog der DE-A-26 09 659 (= US 4,102,701), veröffentlicht am 30. September 1976.

In Formel (III) bedeutet R₇ vorzugsweise einen zwei- bis sechswertigen, insbesondere einen zwei-, drei- oder vierwertigen aliphatischen Rest mit 2 bis 10 Kohlenstoffatomen, einen entsprechenden cycloaliphatischen oder aromatischen Rest mit 5 bis 10 Ringkohlenstoffatomen oder einen araliphatischen Rest mit 5 bis 20 Ringkohlenstoffatomen. Gegebenenfalls können diese Reste zusätzlich auch Heteroatome aufweisen. Die Reste R₇ stellen dabei die Rümpfe von Polyalkoholen bzw. Polyolen dar, an denen Hydroxylgruppen in einer Zahl sitzen, die einer der oben angegebenen Wertigkeiten entspricht. Besonders bevorzugt sind Reste R₇, die sich von gerad- und verzweigtkettigen aliphatischen Polyolen, ableiten, z. B. von Glycolen, wie Ethylen- oder Propylenglycol, von Glycerin, Trimethylolpropan, Erythrit oder Pentaerythrit. Ebenfalls bevorzugt als Polyole sind Bisphenoltypen, z. B. 2,2-Di-(4-Hydroxyphenyl)propan oder Di-(4-Hydroxyphenyl)methan, und ähnliche ganz oder teilweise gesättigte Verbindungen, z. B. 2,2-Di-(4-Hydroxycyclohexyl)propan. Ein weiteres Beispiel ist Sorbit. Gegebenenfalls können die Polyole auch dimerisiert bzw. präpolymerisiert sein, es kann sich z. B. also um Polyetheralkohole handeln, wie Polyethylenglycole oder Bis-trimethylolpropan. Ein Polymerisationsgrad von 2 bis 6 ist bei den Präpolymeren bevorzugt. Der Kohlenstoffsechsring in Formel (V) kann entweder aromatisch oder cycloaliphatisch sein, wobei er in letzterem Fall ganz oder nur teilweise gesättigt sein kann. Gegebenenfalls kann er weitere Substituenten, z. B. Chlor, Brom oder C₁-C₄-Alkyl, aufweisen. Dabei sind, je nachdem wie hoch der Sättigungsgrad des Rings ist, bis zu 10 Substituenten am Ring möglich; aus praktischen Gründen ist aber eine Zahl von höchstens 4 zweckmässig. Ganz besonders bevorzugt weist der Ring jedoch nur die Glycidylestergruppen auf. Die einzelnen Reste Z in Formel (III) können auch verschieden sein. Sie müssen auch nicht die gleiche Zahl Glycidylesterreste aufweisen.

Die Verbindungen der Formel (III) sind z. B. in folgender Weise zugänglich. Zunächst wird der gewählte Polyalkohol mit Phthalsäure-, Tetrahydrophthalsäure-, Hexahydrophthalsäure- oder Trimellitsäureanhydrid im entsprechenden stöchiometrischen Verhältnis zum Halbester umgesetzt. Dabei reagiert im Falle von Trimellitsäureanhydrid, das neben der Anhydridgruppe eine Carboxylgruppe aufweist, praktisch nur die Anhydridgruppe zu einem Halbester mit noch zwei freien Carboxylgruppen ab. Die Carboxylgruppen können danach mit Epihalogenhydrin, insbesondere Epichlorhydrin, glycidylisiert werden, wie oben für die Verbindungen der Formel (II) beschrieben.

Soweit Verbindungen der Formel (II) und (III) fest sind, wie es insbesondere für manche Verbindungen der Formel (III) mit zwei Glycidylestergruppen zutrifft, z. B. für den glycidylisierten Halbester aus hydriertem Bisphenol A und Phthalsäure, können die Verbindungen natürlich auch als feste Polyglycidylverbindungen in erfindungsgemässen Zusammensetzungen eingesetzt werden.

Als fliessfähige Polyglycidylverbindungen kommen weiterhin Tri- oder Tetraglycidylether der Formel VII in Frage: 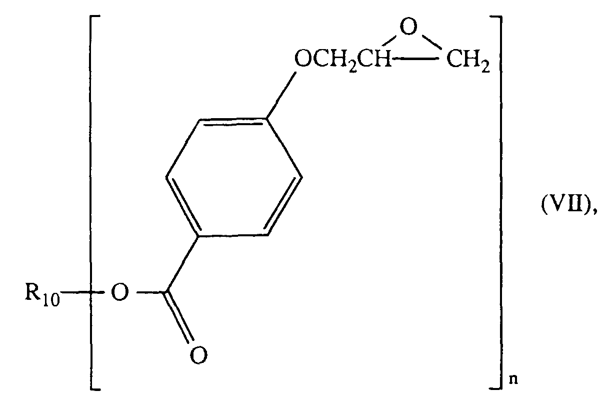 worin n die Werte 3 und 4 annehmen kann und R₁₀ ein organisches Tri- oder Tetraradikal mit 2 bis 15 Kohlenstoffatomen bedeutet.

Bevorzugt handelt es sich bei R₁₀ um Radikale, die sich von Tri- oder Tetraolen ableiten, die ebenfalls als Ausgangsstoffe für Polyester üblich sind, insbesondere um Gruppen der Formeln: 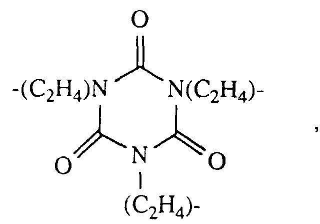 (-CH₂)₃C(C₂H₅) und (-CH₂)₄C.

Die Verbindungen sind in der gleichen Weise erhältlich, wie oben bereits für die entsprechenden Diglycidylether beschrieben, und sie sind ebenfalls teilweise neu.

Obwohl es sich bei den festen und normalerweise in flüssiger Form vorliegenden Polyglycidylverbindungen, aus denen die erfindungsgemässen Zusammensetzungen bestehen, im Regelfall um Verbindungen mit unterschiedlicher chemischer Formel handelt, muss dies nicht unbedingt der Fall sein. Erfindungsgemässe Zusammensetzungen sind z. B. auch erhältlich aus einer bestimmten festen Polyglycidylverbindung und einem Anteil einer Substanz, deren Hauptanteil zwar die gleiche chemische Formel wie die feste Glycidylverbindung aufweist, die jedoch aufgrund von Verunreinigungen bei Raumtemperatur fliessfähig ist. Eine Zusammensetzung unter Anwendung dieses speziellen Prinzips der Erfindung, wird beispielsweise aus festem Trimesinsäuretriglycidylester, der z. B. durch eine Umsetzung von Trimesinsäure oder eines Trimesinsäureesters und Glycidol (z. B. gemäss der DE-C-26 02 157) gewonnen wurde, und aus einer bei Raumtemperatur fliessfähigen Form von Trimesinsäuretriglycidylester, die z. B. aus Trimesinsäure und Epichlorhydrin (gemäss der DE-B-16 43 777) gewonnen werden kann, erhalten.

Der Höchstgehalt an normalerweise in einer flüssigen Form vorliegenden Polyglycidylverbindungen, den die erfindungsgemässen Zusammensetzungen aufweisen können, kann je nach den verwendeten festen und flüssigen Polyglycidylkomponenten und deren Löslichkeit ineinander in weiten Grenzen schwanken. Er ist im Einzelfall aber vom Fachmann mit Hilfe von ein, zwei Orientierungsversuchen leicht festzustellen. Sehr häufig beträgt dieser Höchstgehalt zumindest 45 Gewichtsprozent. Eine bevorzugte Ausführungsform erfindungsgemässer Zusammensetzungen weist daher insgesamt 5 bis 45 Gewichtsprozent der Polyglycidylverbindungen auf, die normalerweise in einer flüssigen Form vorliegen, wobei sich die Gehaltsangabe auf die Gesamtmenge aller Polyglycidylverbindungen in der Zusammensetzung bezieht. Ganz besonders bevorzugt ist ein Bereich von 15 bis 35 Gewichtsprozent für den Gehalt an flüssigen Polyglycidylverbindungen in den Zusammensetzungen.

Bevorzugt weisen die erfindungsgemässen Zusammensetzungen einen Epoxidgehalt von mehr als 4,5 Äquivalenten Epoxidgruppen pro Kilogramm der Zusammensetzung auf. Besonders bevorzugt sind Zusammensetzungen mit einem Epoxidwert von 6 und mehr Äquivalenten.

Die erfindungsgemässen Zusammensetzungen sind zumindest bei Raumtemperatur (20 bis 25 °C) und tieferen Temperaturen fest und klebfrei. Bevorzugt liegt ihr Erweichungsbereich zwischen 40 und 250 °C, insbesondere zwischen 60 und 120 °C.

Bei einer speziellen Ausführungsform der erfindungsgemässen Zusammensetzungen sind die festen Polyglycidylverbindungen difunktionell und die normalerweise in flüssiger Form vorliegenden Polyglycidylverbindungen zumindest trifunktionell. Zusammensetzungen dieser Art zeigen beispielsweise Härtungseigenschaften, die im wesentlichen denen von Triglycidylisocyanurat ähneln. Dies ist besonders deshalb wichtig, weil die eingangs erwähnte Sonderstellung des Triglycidylisocyanurats unter den Polyglycidylverbindungen dazu geführt hat, dass Reaktionspartner für Glycidylhärter immer mehr für die Verwendung speziell von Triglycidylisocyanurat als Vernetzungsmittel optimiert wurden, so dass heute für manche Einsatzgebiete praktisch nur noch derartige Komponenten kommerziell verfügbar sind. Als Konsequenz der Optimierung zeigen diese Komponenten aber beispielsweise allein mit Diglycidylverbindungen als Härter im allgemeinen nur noch schlechte Eigenschaften.

Besonders bevorzugte Zusammensetzungen der zuletzt genannten Art sind die, die eine feste Lösung aus 60 bis 85, insbesondere 75 bis 85 Gewichtsprozent Diglycidylterephthalat und insgesamt 15 bis 40, insbesondere 15 bis 25 Gewichtsprozent Trimellitsäuretriglycidylester und/oder fliessfähigem Trimesinsäuretriglycidylester darstellen, z. B. eine Zusammensetzung aus 75 Gewichtsprozent Terephthalsäurediglycidylester und 25 Gewichtsprozent Trimellitsäuretriglycidylester.

Die vorstehend beschriebenen festen Zusammensetzungen aus Polyglycidylverbindungen als wesentlichen Bestandteilen, sind im allgemeinen durch ein Verfahren erhältlich, in dessen Verlauf ein oder mehrere feste Polyglycidylverbindungen zusammen mit insgesamt nicht weniger als 5 Gewichtsprozent, bezogen auf die Gesamtmenge an Polylycidylverbindungen, einer Polyglycidylverbindung oder eines Gemisches mehrerer Polyglycidylverbindungen, die in flüssiger Form vorliegen, erhitzt werden, bis die genannten Polyglycidylverbindungen eine im wesentlichen homogene, flüssige Mischung bilden, und die Temperatur im weiteren Verlauf des Verfahrens zumindest soweit abgesenkt wird, dass das feste Produkt entsteht.

Ein bei dem vorstehend beschriebenen Verfahren erhaltenes festes Produkt wird schliesslich bevorzugt noch granuliert. Die nach einer Granulierung erhaltenen Pulverpartikel backen und kleben bei üblichen Lagerungstemperaturen (Raumtemperatur oder mässig erhöhte Temperatur) nicht zusammen und sind auch sonst physikalisch und chemisch lagerstabil.

Es ist aber z. B. auch möglich, die für die Zusammensetzung vorgesehenen festen und flüssigen Polyglycidylverbindungen in geeigneten Lösungsmittels zu lösen und aus den vereinigten Lösungen alles Lösungsmittel soweit zu entfernen, dass die erfindungsgemässen festen Zusammensetzungen erhalten werden können.

Ebenso können beispielsweise bestimmte Verbindungen gemeinsam glycidyliert werden, wie etwa Polycarbonsäuren und/oder Polycarbonsäureanhydride, die Polyglycidylester bilden, die normalerweise in flüssiger Form vorliegen, im Gemisch mit einer genügenden Menge von Polycarbonsäuren und/oder Polycarbonsäureanhydride, die feste Glycidylester bilden. Diese besonders einfache Art, erfindungsgemässe Zusammensetzungen, die aus Polyglycidylestern bestehen, herzustellen, ist besonders bevorzugt. Die Erfindung betrifft daher auch ein Verfahren zur Herstellung erfindungsgemässer Zusammensetzungen, die aus Polyglycidylestern als Polyglycidylverbindungen bestehen, bei dem eine oder mehrere Polycarbonsäuren und/oder Polycarbonsäureanhydride, die feste Polyglycidylester bilden, im Gemisch mit einer oder mehreren Polycarbonsäuren und/oder Polycarbonsäureanhydriden, die Polyglycidylester bilden, die normalerweise in flüssiger Form vorliegen, zu Glycidylestern überführt werden, wobei die Mengen der Polycarbonsäuren so gewählt sind, dass eine oder mehrere feste Mischphasen entstehen, die im wesentlichen die Gesamtmenge der normalerweise in flüssiger Form vorliegenden Polyglycidylverbindungen als zusätzliche Komponente oder Komponenten enthält oder enthalten.

Das genannte Verfahren ist z. B. gut geeignet zur Herstellung erfindungsgemässer Zusammensetzungen auf Basis von Terephthalsäurediglycidylester, Isophthalsäurediglycidylester, Trans-hexahydrophthalsäurediglycidylester, Trimellitsäuretriglycidylester, Trimesinsäuretriglycidylester und Pyromellitsäuretetraglycidylester.

Die gemeinsame Überführung des Gemisches aus Polycarbonsäuren in die entsprechenden Polyglycidylester kann prinzipiell nach jedem hierfür geeigneten Verfahren erfolgen. Bevorzugt ist aber eine Verfahrensweise gemäss der Deutschen Auslegeschrift DE-B- 1 643 777 (ausgelegt am 14. Juni 1973), auf die hier nur verwiesen wird, deren Offenbarung aber als Bestandteil dieser Beschreibung angesehen wird. Das Verfahren aus der DE-B-1 643 777 eignet sich z. B. hervorragend zur Herstellung erfindungsgemässer Zusammensetzungen auf Basis von 60 bis 85 Gewichtsprozent Diglycidylterephthalat und insgesamt 15 bis 40 Gewichtsprozent Trimellitsäuretriglycidylester und/oder fliessfähigem Trimesinsäuretriglycidylester, z. B. von etwa 75 Gewichtsprozent Diglycidylterephthalat und etwa 25 Gewichtsprozent Trimesinsäuretriglycidylester.

Die erfindungsgemässen Zusammensetzungen eignen sich unter anderem als Vernetzungsmittel bzw. als Härter für Substanzen, die funktionelle Gruppen aufweisen, die mit Epoxidgruppen reagieren, z. B. Hydroxyl-, Thiol-, Amino-, Amid- oder insbesondere Carboxylgruppen. Weitere Beispiele geeigneter funktioneller Gruppen sind in Henry Lee, Kris Neville, "Handbook of Epoxy Resins", MacGraw-Hill, Inc. 1967, Appendix 5-1 beschrieben. Bei manchen funktionellen Gruppen kann der Zusatz eines Katalysators zweckmässig sein. Gemische dieser Art können im allgemeinen bei Temperaturen zwischen 100 und 250 °C ausgehärtet werden und sind vielseitig, z. B. als Schmelzklebstoffe, Giessharze oder Pressmassen, einsetzbar. Bevorzugt ist die Verwendung als Vernetzungsmittel für solche epoxidgruppenhaltigen Substanzen, die bei Raumtemperatur oder mässig erhöhter Temperatur fest sind.

Ein besonders bevorzugtes Anwendungsgebiet der erfindungsgemässen Zusammensetzungen sind Pulverlackapplikationen. Hierbei können erfindungsgemässe Zusammensetzungen beispielsweise das in dieser Technologie besonders weit verbreitete und im allgemeinen mehr toxische Triglycidylisocyanurat ersetzen, für gewöhnlich ohne dass wesentliche Veränderungen bei den übrigen Komponenten der Pulverlacke oder der Herstellung der Lacke erforderlich wären und lacktechnische Nachteile einkalkuliert werden müssten.

Einen weiteren Gegenstand der Erfindung stellen daher Pulverlacke, insbesondere auf Basis von Polyestern mit freien Carboxylgruppen dar, die eine der oben beschriebenen Zusammensetzungen enthalten und diese als Vernetzungsmittel verwenden.

Vorzugsweise basieren diese Pulverlacke auf in dieser Technologie üblicherweise verwendeten Polyestern mit endständigen Carboxylgruppen. Die Polyester weisen bevorzugt eine Säurezahl (angegeben in mg KOH/g Polyester) von 10 bis 100 und einem Molekulargewicht von 500 bis 10000, bevorzugt bis 2000, auf. Die verwendeten Polyester sind zweckmässig bei Raumtemperatur fest und haben eine Glasumwandlungstemperatur von 35 bis 120 °C, vorzugsweise von 40 bis 80 °C.

Polyester, wie im vorigen Abschnitt beschrieben, sind z. B. aus der US-A-3,397,254 bekannt. Sie sind Reaktionsprodukte von Polyolen mit Dicarbonsäuren und gegebenenfalls polyfunktionellen Carbonsäuren. Geeignete Polyole sind beispielsweise Ethylenglycol, Propylenglycol, 1,3-Butandiol, 1,4-Butandiol, Neopentandiol, Isopentylglycol, 1,6 Hexandiol, Glycerin, Trimethylolethan, Trimethylolpropan, Erythrit, Pentaerythrit oder Cyclohexandiol. Insbesondere Neopentandiol stellt einen wesentlichen Bestandteil der Polyesterharze dar, die für hochwetterfeste Anstriche geeignet sind. Als Dicarbonsäuren geeignet sind z. B. Isophthalsäure, Terephthalsäure, Phthalsäure, Methylphthalsäuren, Tetrahydrophthalsäure, Methyltetrahydrophthalsäuren, z. B. 4-Methyltetrahydrophthalsäure, Cyclohexandicarbonsäuren, Bernsteinsäure, Glutarsäure, Adipinsäure, Pimelinsäure, Suberinsäure, Azelainsäure, Sebacinsäure, Fumarsäure, Maleinsäure oder 4,4'-Diphenyldicarbonsäure, usw.. Geeignete Tricarbonsäureanhydride sind z. B. die Anhydride von aliphatischen Tricarbonsäuren, wie 1,2,3-Propantricarbonsäure, von aromatischen Tricarbonsäuren, wie Trimellitsäure (Benzol-1,2,4-tricarbonsäure) und Hemimellitsäure (Benzol-1,2,3-tricarbonsäure), oder von cycloaliphatischen Tricarbonsäuren, wie 6-Methyl-cyclohex-4-en-1,2,3-tricarbonsäure. Geeignete Tetracarbonsäureanhydride sind z. B. Pyromellitsäuredianhydrid oder Benzophenon-3,3',4,4'-tetracarbonsäuredianhydrid.

Die erfindungsgemässen Zusammensetzungen werden dabei bevorzugt in einer solchen Menge eingesetzt, dass das Verhältnis Carboxylgruppen zu Epoxidgruppen im Pulverlack zwischen 0,5 :1 und 2 : 1 liegt.

Die Pulverlacke enthalten selbstverständlich im allgemeinen noch weitere in der Lackindustrie übliche Zusatzstoffe, wie beispielsweise Lichtschutzmittel, Farbstoffe, Pigmente, z. B. Titandioxidpigment, Entgasungsmittel, z. B. Benzoin, und/oder Verlaufsmittel. Geeignete Verlaufsmittel sind z. B. Polyvinylacetale, wie Polyvinylbutyral, Polyethylenglycol, Polyvinylpyrrolidon, Glycerin, Acrylmischpolymerisate, wie sie z. B. unter den Bezeichnungen Modaflow® [MONSANTO] oder Acrylron® [PROTEX] erhältlich sind.

Erfindungsgemässe Pulverlacke können durch einfaches Mischen der Bestandteile, z. B. in einer Kugelmühle, hergestellt werden. Eine andere und mehr bevorzugte Möglichkeit besteht darin, dass man die Bestandteile zusammen aufschmilzt, vermischt und homogenisiert, vorzugsweise in einer Extrusionsmaschine, wie einem Buss-Kokneter, die Masse abkühlt und zerkleinert. Die Pulverlackmischungen weisen vorzugsweise eine Partikelgrösse im Bereich von 0,015 bis 500 µm, insbesondere von 10 bis 75 µm, auf.

Die Pulverlacke werden nach Applikation auf den zu beschichtenden Gegenstand bei einer Temperatur von mindestens etwa 100 °C, vorzugsweise 150 bis 250 °C, gehärtet. Für die Härtung sind im allgemeinen etwa 5 bis 60 Minuten erforderlich. Zur Beschichtung geeignet sind alle Materialien, die bei den zur Härtung erforderlichen Temperaturen stabil sind, insbesondere Keramik und Metalle.

Insbesondere bei Verwendung von Polyestern, die zu mehr als 50, insbesondere zu 90 und mehr Gew.-%, aus Neopentandiol und aromatischen Dicarbonsäuren, insbesondere Terephthalsäure, als Baueinheiten bestehen und z. B. als Crylcoat®-Typen [UCB] oder unter Bezeichnungen wie Uralac® [DSM] oder Grilesta® [EMS] im Handel sind, werden Pulverlacke erhalten, die witterungsstabile, für Aussenanstriche geeignete und besonders flexible Beschichtungen ergeben, was sowohl für plötzliche als auch für andauernde mechanische Belastung zutrifft.

Beispiel 1: 100 g Trimellitsäuretriglycidylester [viskoses Produkt mit einem Epoxidgehalt von 6,91 Epoxidäquivalente/kg (Theorie: 7,92 Epoxidäquivalente/kg), hergestellt gemäss DE-A-16 43 777, Beispiel 15, veröffentlicht am 8. Juni 1972] werden mit 400 g Terephthalsäurediglycidylester [festes Produkt mit einem Schmelzbereich von 97-103 °C und einem Epoxidgehalt von 6,40 Epoxidäquivalente/kg (89% der Theorie), hergestellt gemäss DE-A-16 43 777, Beispiel 7] gemischt. Die Temperatur der Mischung wird auf 105 °C erhöht, bis die Lösung klar wird. Dann wird das Gemisch abgekühlt. Dabei bildet sich quantitativ ein farbloses, kristallines und nicht klebriges Produkt mit einem Schmelzpunkt von 92 °C und einem Epoxidgehalt von 6,75 Epoxidäquivalente/kg.

Beispiel 2: 125 g des in Beispiel 1 verwendeten Trimellitsäuretriglycidylesters werden mit 375 g des in Beispiel 1 verwendeten Terephthalsäurediglycidylesters gemischt. Die Temperatur der Mischung wird auf 105 °C erhöht, bis die Lösung klar wird. Dann wird das Gemisch abgekühlt. Dabei bildet sich quantitativ ein farbloses, kristallines und nicht klebriges Produkt mit einem Schmelzpunkt von 83 °C und einem Epoxidgehalt von 6,83 Epoxidäquivalente/kg.

Beispiel 3: 100 g 2,2,6,6-Tetra-(β-carboxyethyl)-cyclohexanontetraglycidylester (Epoxidgehalt von 5,29 Epoxidäquivalente/kg, hergestellt gemäss DE-A-23 19 815) werden mit 300 g Terephthalsäurediglycidylester [festes Produkt mit einem Schmelzbereich von 97-103 °C und einem Epoxidgehalt von 6,40 Epoxidäquivalente/kg (89% der Theorie), hergestellt gemäss DE-A-16 43 777, Beispiel 7] gemischt. Die Temperatur der Mischung wird auf 105 °C erhöht, bis die Lösung klar wird. Dann wird das Gemisch abgekühlt. Dabei bildet sich quantitativ ein farbloses, kristallines und nicht klebriges Produkt mit einem Schmelzpunkt von 83 °C und einem Epoxidgehalt von 6,32 Epoxidäquivalente/kg.

Beispiel 4: Herstellung und Glycidylierung des Bis-(4-hydroxybenzoesäure)-esters von 1,6-Hexandiol.

413,6 g (3,5 Mol) 1,6-Hexandiol werden bei einer Temperatur von ca. 60 °C geschmolzen.

Es werden 1065,05 g (7 Mol) 4-Hydroxybenzoesäuremethylester und 2,95 g Tetrabutylorthotitanat zugegeben. Anschliessend wird unter Stickstoff während 4 Stunden auf 210 °C erhitzt. Es werden 1254,7 g des Bis-(4-hydroxybenzoesäure)-esters von 1,6-Hexandiol in Form eines festen Produkts (Erweichungspunkt: 176 °C) entsprechend einer Massenausbeute von 99,8 % mit einem Phenolgehalt von 5,52 Phenoläquivalenten/kg ( = 99 % der Theorie) erhalten.

1000 g dieses Produkts werden mit 3574 g (38,6 Mol) Epichlorhydrin versetzt, und die Temperatur wird auf 90 °C gehalten. Es werden 32,24 g einer 50%-igen wässerigen Lösung von Tetramethylammoniumchlorid zugegeben. Die Temperatur wird zwischen 85 und 90 °C gehalten, und der Verlauf der Reaktion wird mit Hilfe einer pH-Elektrode überwacht. Nach etwa 150 min steigt die Anzeige am pH-Messgerät sprunghaft auf einen Wert von ca. 9,4 an, was das Ende der Anlagerungsreaktion anzeigt. Nach Entfernung der pH-Elektrode wird das Reaktionsgemisch auf 50 °C gekühlt. Unter einem Vakuum von 0,09 bis 0,13 bar und bei einer Innentemperatur von 45 bis 50 °C werden während 300 min 485,7 g wässerige 50%-ige Natronlauge kontinuierlich zulaufen lassen, wobei das eingebrachte und gebildete Wasser mit Epichlorhydrin als azeotropes Gemisch abdestillieren. Das in einem Wasserabscheider vom Wasser abgetrennte Epichlorhydrin wird kontinuierlich ins Reaktionsgemisch zurückgeführt. Danach wird das Reaktionsgemisch mit 400 ml wässeriger 10 %-iger Mononatriumphosphatlösung und dreimal mit 500 ml Wasser gewaschen. Man erhält 1089,4 g (84 % der Theorie) des Bis-(4-glycidyloxybenzoesäure)-esters von 1,6-Hexandiol in Form eines festen Produkts (Schmelzpunkt 120 °C, Epoxidgehalt 4,24 Äquivalente/kg, entsprechend 100% der Theorie, Chlorgehalt 0,15%).

Beispiel 5: 260 g des Produkts aus Beispiel 4 werden mit 140 g Trimellitsäuretriglycidylester gemischt. Die Temperatur der Mischung wird auf 130 °C erhöht, bis die Lösung klar wird. Dann wird das Gemisch abgekühlt. Dabei bildet sich quantitativ ein farbloses, kristallines Produkt mit einem Schmelzpunkt von 115 °C und einem Epoxidgehalt von 5, 12 Epoxidäquivalente/kg.

Beispiel 6: In einem Extruder (Kokneter der Firma Buss, Pratteln, CH) wird bei einer Temperatur zwischen 30 und 110 °C, vorzugsweise zwischen 70 und 90 °C, ein Gemisch aus 930 g Crylcoat® 430 [carboxylterminierter Polyester auf Basis von Neopentandiol und Terephthalsäure mit einer Säurezahl von ca. 30 mg KOH/g und einer Glasumwandlungstemperatur (T_{G}) von ca. 70 °C (DSC), hergestellt von UCB, Belgien], 97 g des Produkts aus Beispiel 1, 11 g Acrylron®fest (Verlaufsmittel auf Basis eines butylierten Polyacrylats), 2 g Benzoin, 30 g eines Katalysators für die Härtung auf Basis eines Konzentrats eines Tetraalkylammoniumbromidsalzes und 500 g Titandioxid homogenisiert. Das abgekühlte Extrudat wird zu einer Partikelgrösse von ca. 40 µm zum fertigen Pulverlack vermahlen.

Dieser Pulverlack wird elektrostatisch auf ein Aluminiumblech aufgesprüht. Nach zehnminütigem Einbrennen bei einer Temperatur von 200 °C wird ein Lackfilm mit folgenden Eigenschaften erhalten:

| | |
|---|---|
| Filmdicke | 55 µm |
| Schlagverformung, rückseitig | 160 kg·cm |
| Erichsentiefziehtest (nach DIN 53156) | 10 mm |
| Glanz unter einem Winkel von 60° | 90 % |
| Verlauf bei 200 °C | gut |
| Gelbwert (nach DIN 6167/ASTM D 1925-70 | 0 |

Die Schlagverformung wird bestimmt, indem man einen Stempel bekannten Gewichts aus bestimmter Höhe von hinten auf die beschichtete Fläche fallen lässt. Der angegebene Wert ist das Produkt aus dem Gewicht des Stempels in kg und der grössten Höhe in cm, bei der die Beschichtung noch unbeschädigt bleibt.

Der Verlauf wird visuell als mässig, gut oder sehr gut abgeschätzt.

Dieses Lacksystem weist eine höhere Reaktivität und Flexibilität auf als ein System auf Basis von reinem Diglycidylterephthalat.

Beispiel 7: Wie in Beispiel 6 beschrieben, wird ein Pulverlack aus 930 g Crylcoat®430, 95,5 g des Produkts von Beispiel 2, 11 g Acrylron®fest, 3 g Benzoin, 30 g eines Katalysators (Konzentrat eines Tetraalkylammoniumbromidsalzes) und 500 g Titandioxid homogenisiert. Der erhaltene Pulverlack wird elektrostatisch auf ein Aluminiumblech aufgesprüht und während 10 Minuten bei 200 °C eingebrannt. Es entsteht ein Lackfilm mit folgenden Eigenschaften:

| | |
|---|---|
| Filmdicke | 56 µm |
| Schlagverformung, rückseitig | 160 kg·cm |
| Erichsentiefziehtest (nach DIN 53156) | 10 mm |
| Glanz unter einem Winkel von 60° | 90 % |
| Verlauf bei 200 °C | gut |
| Gelbwert (nach DIN 6167/ASTM D 1925-70) | 0 |

Dieser Pulverlack weist eine höhere Reaktivität und Flexibilität auf als ein System auf Basis von reinem Diglycidylterephthalat.

Beispiel 8: Wie in Beispiel 6 beschrieben, wird ein Pulverlack aus 930 g Crylcoat®430, 103,5 g des Produkts von Beispiel 3, 11 g Acrylron®fest, 3 g Benzoin, 30 g eines Katalysators (Konzentrat eines Tetraalkylammoniumbromidsalzes) und 500 g Titandioxid homogenisiert. Der erhaltene Pulverlack wird elektrostatisch auf ein Aluminiumblech aufgesprüht und während 15 Minuten bei 200 °C eingebrannt. Es entsteht ein Lackfilm mit folgenden Eigenschaften:

| | |
|---|---|
| Fimdicke | 55 µm |
| Schlagverformung, rückseitig | 160 kg·cm |
| Erichsentiefziehtest (nach DIN 53156) | 10 mm |
| Glanz unter einem Winkel von 60° | 94 % |
| Verlauf bei 200 °C | gut |
| Gelbwert (nach DIN 6167/ASTM D 1925-70 | 0 |

Dieser Pulverlack weist eine höhere Reaktivität und Flexibilität auf als ein System auf Basis von reinem Diglycidylterephthalat.

Beispiel 9: Wie in Beispiel 6 beschrieben, wird ein Pulverlack aus 930 g Crylcoat®430, 120 g des Produkts von Beispiel 4, 12 g Acrylron®fest, 3 g Benzoin, 30 g eines Katalysators (Konzentrat eines Tetraalkylammoniumbromidsalzes) und 525 g Titandioxid homogenisiert. Der erhaltene Pulverlack wird elektrostatisch auf ein Aluminiumblech aufgesprüht und während 10 Minuten bei 200 °C eingebrannt. Es entsteht ein Lackfilm mit folgenden Eigenschaften:

| | |
|---|---|
| Filmdicke | 57 µm |
| Schlagverformung, rückseitig | 140 kg·cm |
| Erichsentiefziehtest (nach DIN 53156) | 10,4 mm |
| Glanz unter einem Winkel von 60° | 93 % |
| Verlauf bei 200 °C | gut |
| Gelbwert (nach DIN 6167/ASTM D 1925-70) | 0 |

Dieser Pulverlack weist eine höhere Reaktivität und Flexibilität auf als ein System auf Basis von reinem Diglycidylterephthalat.

Beispiel 10: 465,6 g (5,03 Mol) Epichlorhydrin und 25,6 g Wasser werden auf eine Temperatur von etwa 60 °C erhitzt. Es werden 30,0 g (0, 156 Mol) Trimellitsäureanhydrid, 100,4 g (0,602 Mol) Terephthalsäure und 5,1 g einer 50 %-igen wässerigen Lösung von Tetramethylammoniumchlorid zugegeben. Die Temperatur wird danach bei etwa 90 °C gehalten und der Verlauf der Reaktion mit Hilfe einer pH-Elektrode überwacht. Nach etwa 120 Minuten steigt der angezeigte pH-Wert sprunghaft auf etwa 9,4 an. Danach wird die pH-Elektrode entfernt und das Reaktionsgemisch auf etwa 15 °C abgekühlt. 310,4 g (3,35 Mol) Epichlorhydrin und 20,6 g einer 50 %-igen wässerigen Lösung von Tetramethylammoniumchlorid werden zugegeben. Unter einem Vakuum von 120 mbar und bei einer Innentemperatur von 50 °C werden während 300 Minuten 154,3 g wässerige Natronlauge kontinuierlich zulaufen lassen, wobei das eingebrachte und gebildete Wasser mit Epichlorhydrin als azeotropes Gemisch abdestillieren. Danach wird das Reaktionsgemisch mit 450 ml Wasser, mit 120 ml wässeriger 5 %-iger Natriumbisulfatlösung und anschliessend mit 200 ml Wasser gewaschen und im Wasserstrahlvakuum eingeengt.

Man erhält 204,4 g (90 % der Theorie) eines festen Produkts (Schmelzpunkt 99,9 °C; Epoxidgehalt 6,86 Äquivalente pro Kilogramm; Chlorgehalt 0,63 %).)

## Patentansprüche

1. Feste Zusammensetzung aus Polyglycidylverbindungen eines Molekulargewichts unter 1500 mit einem Epoxidgehalt von über 3,5 Äquivalenten pro Kilogramm Verbindung, die aus einer oder mehreren festen Polyglycidylverbindungen und insgesamt nicht weniger als 5 Gewichtsprozent einer normalerweise in flüssiger Form vorliegenden Polyglycidylverbindung oder eines Gemisches von normalerweise in flüssiger Form vorliegenden Polyglycidylverbindungen als wesentlichen Komponenten besteht, wobei sich die Gehaltsangabe auf die Gesamtmenge aller Polyglycidylverbindungen in der Zusammensetzung bezieht und die Zusammensetzung die festen Polyglycidylverbindungen oder zumindest einen Teil der festen Polyglycidylverbindungen in Form einer oder mehr als einer festen Mischphase enthält und die feste Mischphase oder die festen Mischphasen im wesentlichen die Gesamtmenge der normalerweise in flüssiger Form vorliegenden Polyglycidylverbindungen als zusätzliche Komponente oder Komponenten enthält oder enthalten.

2. Zusammensetzung nach Anspruch 1. bei der es sich um eine feste, kristalline Mischphase (feste Lösung) aus einer festen und einer weiteren Polyglycidylverbindung, die normalerweise in einer flüssigen Form vorliegt, handelt.

3. Zusammensetzung nach einem der Ansprüche 1 oder 2, die aus Polyglycidylverbindungen besteht. die Glycidylether- und/oder Glycidylestergruppen aufweisen.

4. Zusammensetzung nach mindestens einem der Ansprüche 1 bis 3, die Diglycidylester und/oder Diglycidylether als feste Polyglycidylverbindungen enthält.

5. Zusammensetzung nach Anspruch 4. die als Diglycidylester Terephthalsäurediglycidylester oder Isophthalsäurediglycidylester oder Trans-hexahydrophthalsäurediglycidylester oder Oxalsäurediglycidylester oder Adipinsäurediglycidylester oder Sebazinsäurediglycidylester oder Azelainsäurediglycidylester oder Bernsteinsäurediglycidylester enthält.

6. Zusammensetzung nach Anspruch 4, die einen Diglycidylether der Formel (I) enthält: 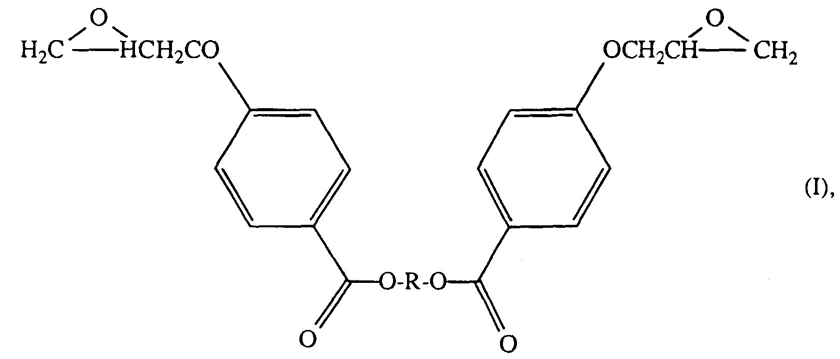 worin R ein organisches Diradikal mit 2 bis 15 Kohlenstoffatomen bedeutet.

7. Zusammensetzung nach mindestens einem der Ansprüche 1 bis 6, die Polyglycidylether und/oder Polyglycidylester mit mindestens drei Glycidylgruppen pro Molekül als normalerweise in einer flüssigen Form vorliegende Polyglycidylverbindungen enthält.

8. Zusammensetzung nach Anspruch 7, bei der die Polyglycidylester mit mindestens drei Glycidylgruppen pro Molekül ausgewählt sind aus nicht festen Formen von Trimellitsäuretriglycidylester, Trimesinsäuretriglycidylester und Pyromellitsäuretetraglycidylester.

9. Zusammensetzungen nach Anspruch 7, bei der die Polyglycidylester mit mindestens drei Glycidylgruppen pro Molekül Verbindungen der Formeln (II) oder (III) sind: 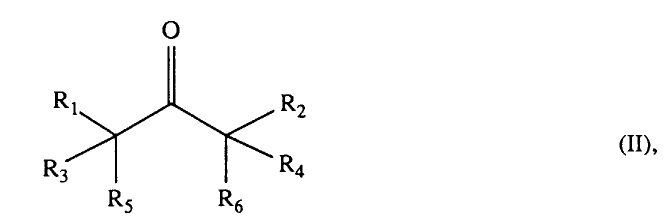 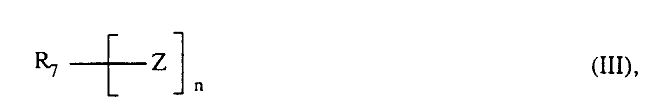 wobei in Formel (II) R₁, R₂, R₃ und R₄ unabhängig voneinander Wasserstoff, C₁-C₄-Alkyl oder Reste der Formel (IV) bedeuten: 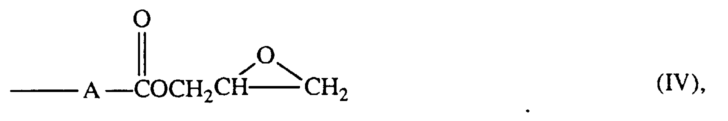 worin A für eine Polymethylengruppe mit 2 bis 4 Kohlenstoffatomen steht, und R₅ und R₆ unabhängig voneinander Wasserstoff, C₁-C₄-Alkyl, Reste der Formel (IV) oder zusammen eine unsubstituierte oder eine mit C₁-C₄-Alkyl substituierte Methylen- oder Polymethylengruppe mit 2 bis 7 Kohlenstoffatomen bedeuten, und in Formel (III) n eine ganze Zahl von 2 bis 6, R₇ einen n-wertigen organischen Rest, und Z gleiche oder unterschiedliche Reste der Formel (V) bedeuten: 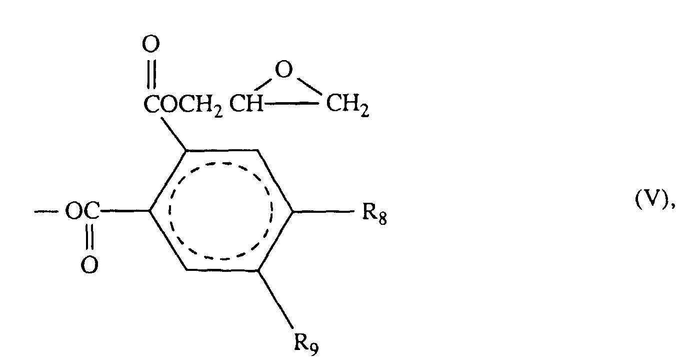 worin R₈ und R₉ entweder unabhängig voneinander Wasserstoff, Chlor, Brom oder C₁-C₄-Alkyl oder eines von beiden einen Rest der Formel (VI): 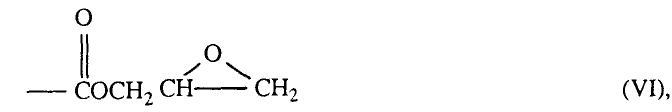 und das andere Wasserstoff, Chlor, Brom oder C₁-C₄-Alkyl bedeuten, und der sechsgliedrige Ring in Formel (V) aromatisch oder nicht aromatisch ist.

10. Zusammensetzung nach Anspruch 7, bei der die Polyglycidylester mit mindestens drei Glycidylgruppen pro Molekül Verbindungen der Formel VII sind: 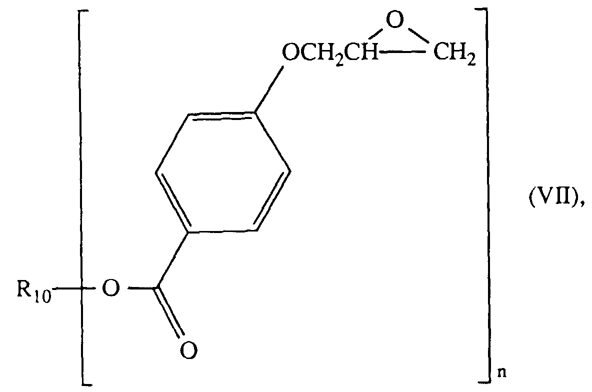 worin n die Werte 3 und 4 annehmen kann und R₁₀ ein organisches Tri- oder Tetraradikal mit 2 bis 15 Kohlenstoffatomen bedeutet.

11. Zusammensetzung nach zumindest einem der Ansprüche 1 bis 10, die insgesamt 5 bis 45 Gewichtsprozent der Polyglycidylverbindungen, die normalerweise in einer flüssigen Form vorliegen, aufweist, wobei sich die Gehaltsangabe auf die Gesamtmenge aller Polyglycidylverbindungen in der Zusammensetzung bezieht.

12. Zusammensetzung nach zumindest einem der Ansprüche 1 bis 11, die einen Epoxidgehalt von mehr als 4,5 Äquivalenten Epoxidgruppen pro Kilogramm der Zusammensetzung, insbesondere von 6 und mehr Äquivalenten, aufweist.

13. Zusammensetzung nach zumindest einem der Ansprüche 1 bis 12, bei der die festen Polyglycidylverbindungen difunktionell und die normalerweise in flüssiger Form vorliegenden Polyglycidylverbindungen zumindest trifunktionell sind.

14. Zusammensetzung nach Anspruch 13, die eine feste Lösung aus 60 bis 85 Gewichtsprozent Diglycidylterephthalat und insgesamt 15 bis 40 Gewichtsprozent Trimellitsäuretriglycidylester und/oder fliessfähigem Trimesinsäuretriglycidylester darstellt.

15. Zusammensetzung nach Anspruch 3, die aus festem Trimesinsäuretriglycidylester und aus einer bei Raumtemperatur fliessfähigen Form von Trimesinsäuretriglycidylester erhalten wurde.

16. Verfahren zur Herstellung von Zusammensetzungen nach mindestens einem der Ansprüche 1 bis 15, in dessen Verlauf ein oder mehrere feste Polyglycidylverbindungen zusammen mit insgesamt nicht weniger als 5 Gewichtsprozent, bezogen auf die Gesamtmenge an Polylycidylverbindungen, einer Polyglycidylverbindung oder eines Gemisches von Polyglycidylverbindungen, die in flüssiger Form vorliegen, erhitzt werden, bis die genannten Polyglycidylverbindungen eine im wesentlichen homogene, flüssige Mischung bilden, und die Temperatur im weiteren Verlauf des Verfahrens zumindest soweit abgesenkt wird, dass das feste Produkt entsteht.

17. Verfahren zur Herstellung von Zusammensetzungen nach mindestens einem der Ansprüche 1 bis 15, die aus Polyglycidylestern als Polyglycidylverbindungen bestehen, bei dem eine oder mehrere Polycarbonsäuren und/oder Polycarbonsäureanhydride, die feste Polyglycidylester bilden, im Gemisch mit einer oder mehreren Polycarbonsäuren und/oder Polycarbonsäureanhydriden, die Polyglycidylester bilden, die normalerweise in flüssiger Form vorliegen, zu Glycidylestern überführt werden, wobei die Mengen der Polycarbonsäuren und/oder Polycarbonsäureanhydride so gewählt sind, dass eine oder mehrere feste Mischphasen entstehen, die im wesentlichen die Gesamtmenge der normalerweise in flüssiger Form vorliegenden Polyglycidylverbindungen als zusätzliche Komponente oder Komponenten enthält oder enthalten.

18. Verwendung einer Zusammensetzung nach mindestens einem der Ansprüche 1 bis 15 als Vernetzungsmittel für Substanzen, die funktionelle Gruppen aufweisen, die mit Epoxidgruppen reagieren.

19. Pulverlack, insbesondere auf Basis von Polyestern mit freien Carboxylgruppen, der eine Zusammensetzung nach mindestens einem der Ansprüche 1 bis 15 als Vernetzungsmittel enthält.

## Claims

1. A solid composition of polyglycidyl compounds having a molecular weight of less than 1500 and an epoxide content of more than 3.5 equivalents per kilogram of compound, which composition consists as essential components of one or more than one solid polyglycidyl compound and altogether not less than 5 per cent by weight of one or of a mixture of more than one polyglycidyl compound, which compound or mixture is normally in liquid form, the indicated content being based on the total amount of all polyglycidyl compounds in the composition, which composition contains the solid polyglycidyl compounds or at least part of the solid polyglycidyl compounds in the form of one or more than one solid mixed phase, which solid mixed phase or solid mixed phases essentially comprises or comprise as additional component or components the total amount of the polyglycidyl compounds which are normally in liquid form.

2. A composition according to claim 1, which is a solid, crystalline mixed phase (solid solution) of a solid polyglycidyl compound and of a further polyglycidyl compound which is normally in liquid form.

3. A composition according to claim 1 or 2, which consists of polyglycidyl compounds which have glycidyl ether and/or glycidyl ester groups.

4. A composition according to at least one of claims 1 to 3, which comprises diglycidyl esters and/or diglycidyl ethers as solid polyglycidyl compounds.

5. A composition according to claim 4, which as diglycidyl esters comprises diglycidyl terephthalate or diglycidyl isophthalate or diglycidyl trans-hexahydrophthalate or diglycidyl oxalate or diglycidyl adipate or diglycidyl sebacate or diglycidyl azelate or diglycidyl succinate.

6. A composition according to claim 4, which comprises a diqlycidyl ether of formula (I) : 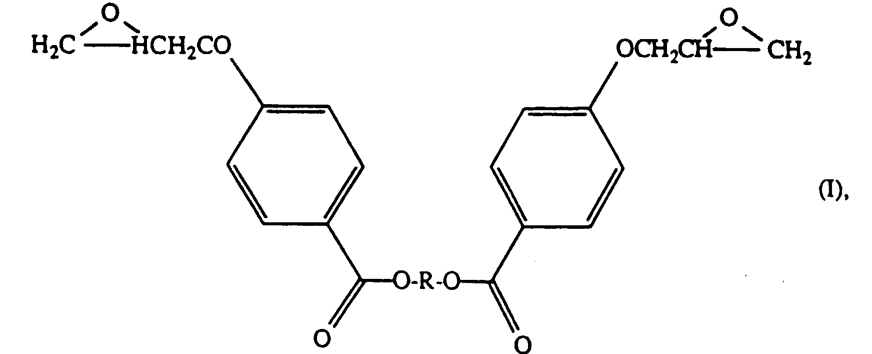 wherein R is an organic divalent radical of 2 to 15 carbon atoms.

7. A composition according to at least one of claims 1 to 6, which comprises polyglycidyl ethers and/or polyglycidyl esters containing at least three glycidyl groups per molecule as polyglycidyl compounds which are normally in liquid form.

8. A composition according to claim 7, wherein the polyglycidyl esters containing at least three glycidyl groups per molecule are selected from non-solid forms of triglycidyl trimellitate, triglycidyl trimesate and tetraglycidyl pyromellitate.

9. A composition according to claim 7, wherein the polyglycidyl esters containing at least three glycidyl groups per molecule are compounds of formula (II) or (III): 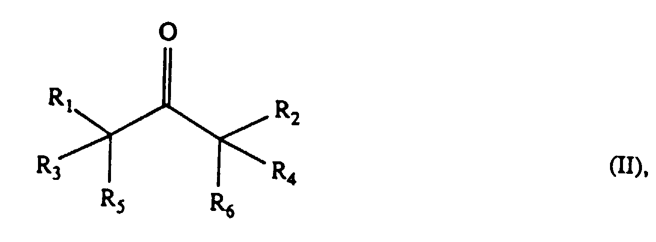 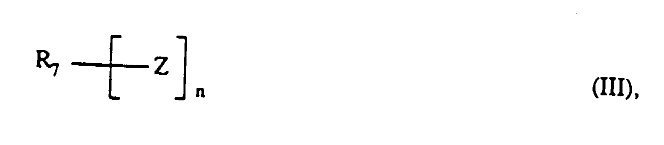 where R₁, R₂, R₃ and R₄ in formula (II) are each independently of one another hydrogen, C₁-C₄alkyl or radicals of formula (IV) : 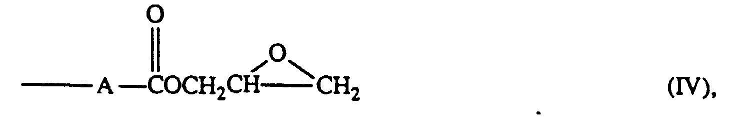 wherein A is a polymethylene group of 2 to 4 carbon atoms, and R₅ and R₆ are each independently of the other hydrogen, C₁-C₄alkyl, radicals of formula (IV) or, taken together, are an unsubstituted or a C₁-C₄alkyl-substituted methylene or polymethylene group of 2 to 7 carbon atoms, and in formula (III) n is an integer from 2 to 6, R₇ is an organic radical of valency n, and the substituents Z are identical or different radicals of formula (V): 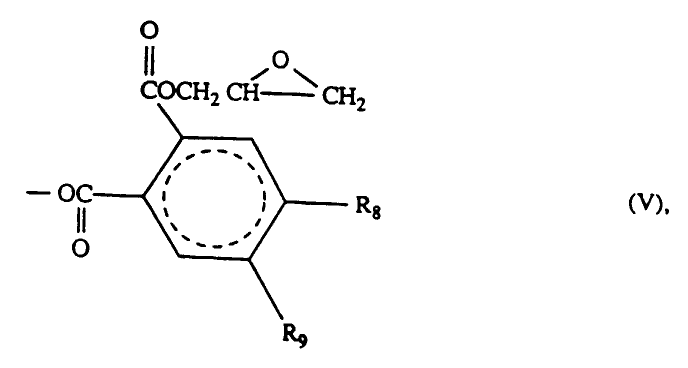 wherein R₈ and R₉ are either each independently of the other hydrogen, chloro, bromo or C₁-C₄alkyl, or one of the two is a radical of formula (VI): 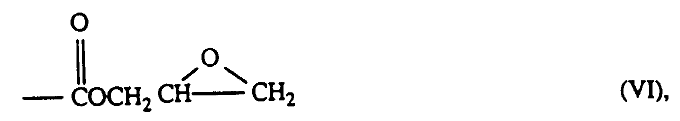 and the other is hydrogen, chloro, bromo or C₁-C₄alkyl, and the six-membered ring in formula (V) is aromatic or nonaromatic.

10. A composition according to claim 7, wherein the polyglycidyl esters containing at least three glycidyl groups per molecule are compounds of formula (VII): 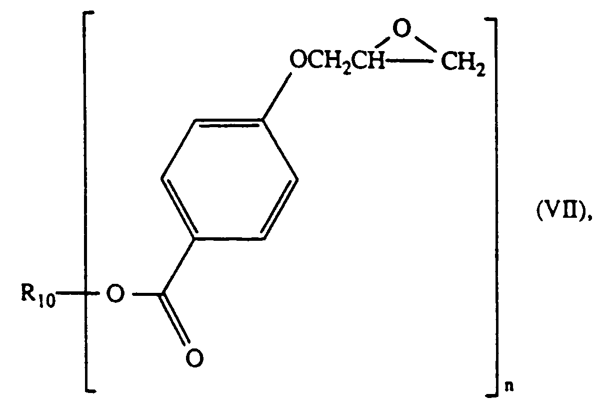 wherein n may be 3 or 4 and R₁₀ is an organic trivalent or tetravalent radical of 2 to 15 carbon atoms.

11. A composition according to at least one of claims 1 to 10, which contains altogether 5 to 45 per cent by weight of polyglycidyl compounds which are normally in liquid form, the indication of content being based on the total amount of all polyglycidyl compounds in the composition.

12. A composition according to at least one of claims 1 to 11, which has an epoxide content of more than 4.5 equivalents of epoxide groups per kilogram of the composition, in particular of 6 or more equivalents.

13. A composition according to at least one of claims 1 to 12, wherein the solid polyglycidyl compounds are bifunctional and the polyglycidyl compounds which are normally in liquid form are at least trifunctional.

14. A composition according to claim 13, which is a solid solution of 60 to 85 per cent by weight of diglycidyl terephthalate and altogether 15 to 40 per cent by weight of triglycidyl trimellitate and/or flowable triglycidyl trimesate.

15. A composition according to claim 3, which has been obtained from solid triglycidyl trimesate and triglycidyl trimesate which is in a flowable form at ambient temperature.

16. A process for the preparation of a composition according to at least one of claims 1 to 15, in the course of which one or more than one solid polyglycidyl compound, together with altogether not less than 5 per cent by weight, based on the total amount of polyglycidyl compounds, of one or of a mixture of more than one polyglycidyl compound, which compound or mixture is in liquid form, is or are heated until the said polyglycidyl compounds form a substantially homogeneous liquid mixture, and the temperature is lowered in the further course of the process at least sufficiently for the solid product to form.

17. A process for the preparation of a composition according to at least one of claims 1 to 15 and consisting of polyglycidyl esters as polyglycidyl compounds, in which process one or more than one polycarboxylic acid and/or polycarboxylic anhydride which forms solid polyglycidyl esters, in admixture with one or more than one polycarboxylic acid and/or polycarboxylic anhydride which forms polyglycidyl esters which are normally in liquid form, is or are converted into glycidyl esters, the amounts of polycarboxylic acids and/or polycarboxylic anhydrides being so chosen that one or more than one solid mixed phase forms, which solid mixed phase or solid mixed phases essentially comprises or comprise as additional component or components the total amount of the polyglycidyl compounds which are normally in liquid form.

18. The use of a composition according to at least one of claims 1 to 15 as a crosslinking agent for substances having functional groups which react with epoxide groups.

19. A powder coating composition, based in particular on polyesters having free carboxyl groups, which comprises as crosslinking agent a composition according to at least one of claims 1 to 15.

## Revendications

1. Composition solide de composés polyglycidyliques d'une masse molaire inférieure à 1500 ayant une teneur en époxyde supérieure à 3,5 équivalents/kg de composé, laquelle est constituée d'un ou de plusieurs composés polyglycidyliques solides et au total de pas moins de 5 % en poids d'un composé polyglycidylique qui se présente normalement sous forme liquide ou d'un mélange de composés de polyglycidyle qui se présente normalement sous forme liquide, en tant que composants principaux, l'indication de la teneur étant relative à la quantité totale de tous les composés polyglycidyliques dans la composition et la composition contient les composés polyglycidyliques solides ou au moins une partie des composés polyglycidyliques solides sous forme d'une ou plus d'une phase mixte solide et la phase mixte solide ou les phases mixtes solides contiennent essentiellement la quantité totale des composés polyglycidyliques qui se présentent normalement sous forme liquide, en tant que composants additionnels.

2. Composition selon la revendication 1, dans laquelle il s'agit d'une phase mixte solide cristalline (solution solide) d'un composé polyglycidylique solide et d'un autre composé polyglycidylique qui se présente normalement sous forme liquide.

3. Composition selon l'une des revendications 1 ou 2, constituée de composés polyglycidyliques qui présentent des groupes éther glycidylique et/ou ester glycidylique.

4. Composition selon au moins l'une des revendications 1 à 3 qui contient des esters diglycidyliques et/ou des éthers diglycidyliques en tant que composés polyglycidyliques solides.

5. Composition selon la revendication 4 qui contient en tant qu'esters diglycidyliques l'ester diglycidylique d'acide téréphtalique ou l'ester diglycidylique d'acide isophtalique ou l'ester diglycidylique d'acide transhexahydrophtalique ou l'ester diglycidylique d'acide oxalique ou l'ester diglycidylique d'acide adipique ou l'ester diglycidylique d'acide sébacique ou l'ester diglycidylique d'acide azélaïque ou l'ester diglycidylique d'acide succinique.

6. Composition selon la revendication 4 qui contient un éther diglycidylique de formule (I) 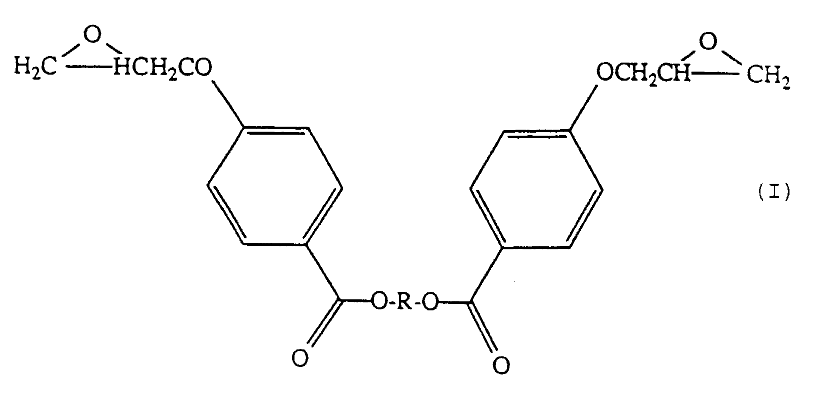 dans laquelle R représente un diradical organique avec 2 à 15 atomes de carbone.

7. Composition selon au moins l'une des revendications 1 à 6, qui contient les éthers polyglycidyliques et/ou les esters polyglycidyliques avec au moins trois groupes glycidyle par molécule en tant que composés polyglycidyliques qui se présentent normalement sous forme liquide.

8. Composition selon la revendication 7, dans laquelle les esters polyglycidyliques avec au moins trois groupes glycidyle par molécule sont pris parmi les formes non solides de l'ester triglycidylique d'acide trimellitique, de l'ester triglycidylique d'acide trimésique et de l'ester tétraglycidylique d'acide pyromellitique.

9. Composition selon la revendication 7 dans laquelle les esters polyglycidyliques avec au moins trois groupes glycidyle par molécule sont des composés de formules (II) ou (III) 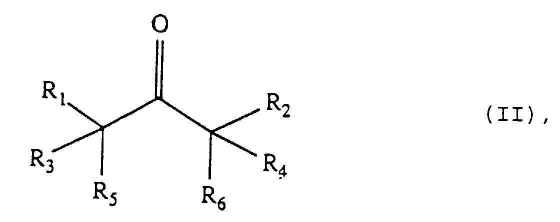 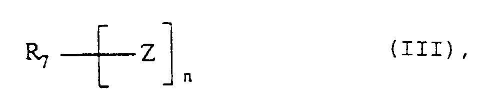 dans la formule (II), R₁, R₂, R₃ et R₄, indépendamment l'un de l'autre, représentant un atome d'hydrogène, un groupe alkyle en C₁-C₄ ou des restes de formule (IV) 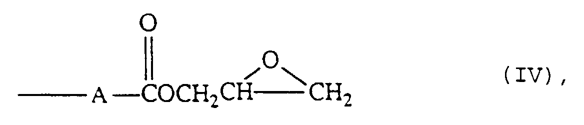 dans laquelle A représente un groupe polyméthylène avec 2 à 4 atomes de carbone, et R₅ et R₆, indépendamment l'un de l'autre, représentent un atome d'hydrogène, des groupes alkyle en C₁-C₄, des restes de formule (IV) ou ensemble représentent un groupe méthylène ou polyméthylène avec 2 à 7 atomes de carbone, non substitués ou substitués par un groupe alkyle en C₁-C₄, et, dans la formule (III), n est un nombre entier de 2 à 6, R₇ représente un reste organique à fonctionnalité n et Z représente des restes identiques ou différents de formule (V) 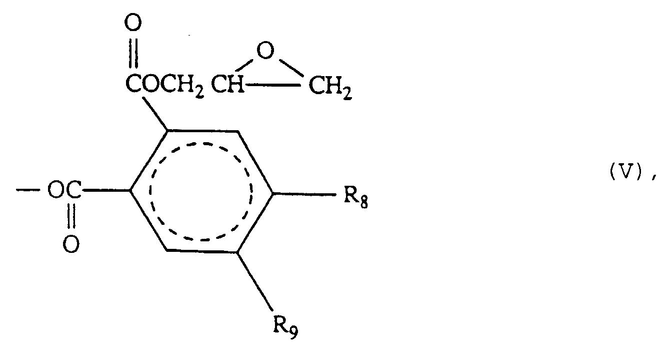 dans laquelle R₈ et R₉ représentent, soit indépendamment l'un de l'autre un atome d'hydrogène, de chlore, de brome ou un groupe alkyle en C₁-C₄, soit l'un des deux restes représente un reste de formule (VI) 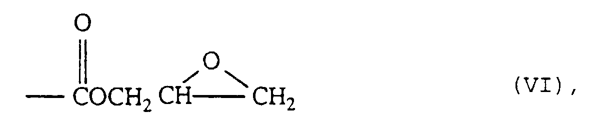 et l'autre représente un atome d'hydrogène, de chlore, de brome ou un groupe alkyle en C₁-C₄, et le cycle à 6 chaînons dans la formule (V) est aromatique ou non aromatique.

10. Composition selon la revendication 7 dans laquelle les esters polyglycidyliques avec au moins trois groupes glycidyle par molécule sont des composés de formule (VII) : 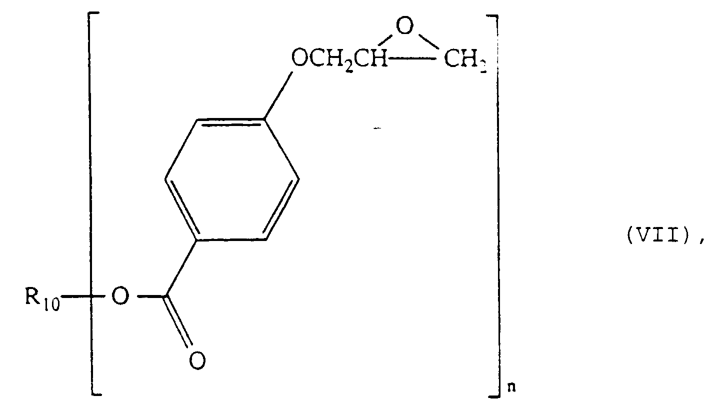 dans laquelle n peut valoir 3 et 4, et R₁₀ représente un radical tri- ou tétrafonctionnel avec 2 à 15 atomes de carbone.

11. Composition selon au moins l'une des revendications 1 à 10 qui contient au total de 5 à 45 % en poids des composés polyglycidyliques qui se présentent normalement sous forme liquide, l'indication de la teneur étant relative à la quantité totale de tous les composés polyglycidyliques de la composition.

12. Composition selon au moins l'une des revendications 1 à 11 qui présente une teneur en époxyde supérieure à 4,5 équivalents de groupes époxyde par kilogramme de la composition, plus particulièrement de 6 et plus de 6 équivalents.

13. Composition selon au moins l'une des revendications 1 à 12, dans laquelle les composés polyglycidyliques solides sont bifonctionnels et les composés polyglycidyliques qui se présentent normalement sous forme liquide, sont au moins trifonctionnels.

14. Composition selon la revendication 13, qui représente une solution solide constituée de 60 à 85 % en poids de téréphtalate de diglycidyle et d'un total de 15 à 40 % en poids d'esters triglycidyliques d'acide trimellitique et/ou d'esters triglycidyliques d'acide trimésique coulables.

15. Composition selon la revendication 3, qui est constituée de l'ester triglycidylique d'acide trimésique solide et de l'ester triglycidylique d'acide trimésique coulable à la température ambiante.

16. Procédé pour la préparation des compositions selon au moins l'une des revendications 1 à 15, dans lequel on chauffe un ou plusieurs composés polyglycidyliques solides conjointement avec pas moins de 5 % en poids au total, par rapport à la quantité totale des composés polyglycidyliques, d'un composé polyglycidylique ou d'un mélange de composés polyglycidyliques, qui se présentent sous forme liquide, jusqu'à ce que les composés polyglycidyliques cités forment un mélange liquide essentiellement homogène, et dans la suite de la réaction, la température diminue pour le moins jusqu'à ce qu'il se forme un produit solide.

17. Procédé pour la préparation des compositions selon au moins l'une des revendications 1 à 15, qui sont constituées d'esters polyglycidyliques en tant que composés polyglycidyliques, dans lequel un ou plusieurs acides polycarboxyliques et/ou anhydrides polycarboxyliques, qui forment des esters polyglycidyliques solides, en mélange avec un ou plusieurs acides polycarboxyliques et/ou anhydrides d'acides polycarboxyliques, qui forment des esters polyglycidyliques qui se présentent normalement sous forme liquide, peuvent être transformés en esters glycidyliques, la quantité des acides polycarboxyliques et/ou anhydrides d'acides polycarboxyliques étant choisie de façon telle qu'il se forme une ou plusieurs phases mixtes solides, qui contiennent essentiellement la quantité totale des composés polyglycidyliques qui se présentent normalement sous forme liquide, en tant que composants supplémentaires.

18. Utilisation d'une composition selon au moins l'une des revendications 1 à 15, en tant qu'agents réticulants pour des substances qui présentent des groupes fonctionnels réagissant avec les groupes époxyde.

19. Vernis en poudre, plus particulièrement à base de polyesters avec des groupes carboxyle libres, qui contient une composition selon au moins l'une des revendications 1 à 15, en tant qu'agent réticulant.
